(19) Europäisches Patentamt
European Patent Office
Office européen des brevets

(11) Publication number: **0 411 787 A2**

(12) **EUROPEAN PATENT APPLICATION**

(21) Application number: **90307830.1**

(51) Int. Cl.⁵: **C12P 17/02**

(22) Date of filing: **18.07.90**

The microorganism(s) has (have) been deposited with NCIMB under number(s) 40155 and 40156.

(30) Priority: **31.07.89 GB 8917496**

(43) Date of publication of application: **06.02.91 Bulletin 91/06**

(84) Designated Contracting States: **AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Seyed-Mahmoudian, Mahmoud**
**44 Weldon Road Marston**
**Oxford OX3 0HP(GB)**
Inventor: **Leak, David Jonathan**
**270 Windmill Road Ealing**
**London W5 4DL(GB)**
Inventor: **Nicholds, Michael John**
**11 The Green High Coniscliffe**
**County Durham DL2 2LJ(GB)**

(74) Representative: **Aufflick, James Neil et al**
**Imperial Chemical Industries PLC Legal Department: Patents PO Box 6 Bessemer Road**
**Welwyn Garden City Herts, AL7 1HD(GB)**

# (54) Microbiological production of 2,3-epoxypropyl ethers.

(57) Microbiological production of 2,3-epoxypropyl ethers, particularly 2,3-epoxypropyl phenyl ethers using the enzyme alkene monooxygenase induced in cells by growth on alkene-containing medium particularly cells derived from the novel strains NCIMB 40155 and NCIMB 40156.

*Fig. 1.*

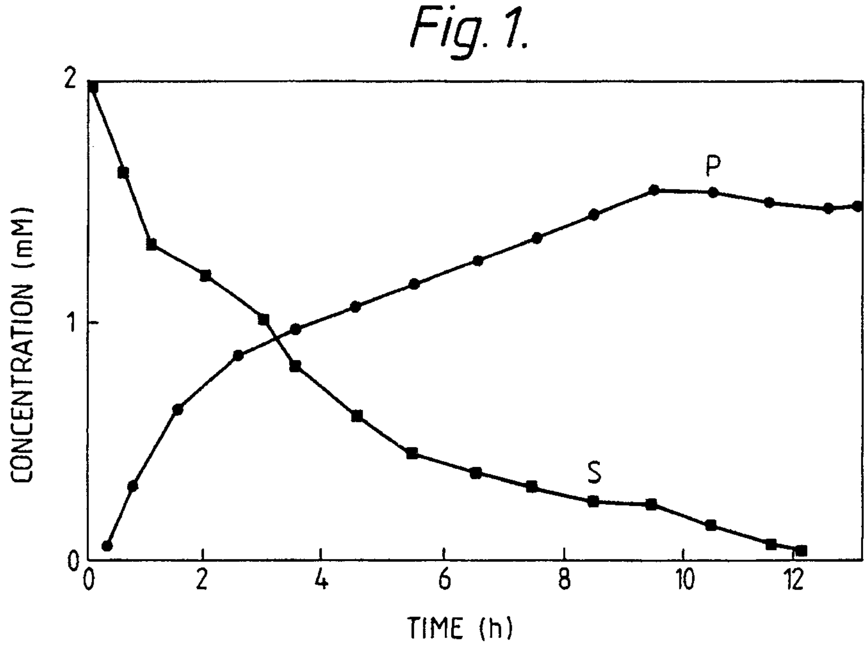




Xerox Copy Centre

## MICROBIOLOGICAL PRODUCTION OF 2,3-EPOXYPROPYL ETHERS

This invention relates to the microbiological production of 2,3-epoxypropyl ethers, in particular 2,3-epoxypropyl phenyl ethers, and to novel microorganisms which are sources of enzymes used in the process.

2,3-epoxypropyl phenyl ethers are optically active compounds which are useful in the agrochemical and pharmaceutical industries, being for example important as starting materials for medicaments. They are important in particular as intermediates in the synthesis of β-blockers since, by ring cleavage using amines, they can be converted into aryloxypropanolamines by the following reaction:-

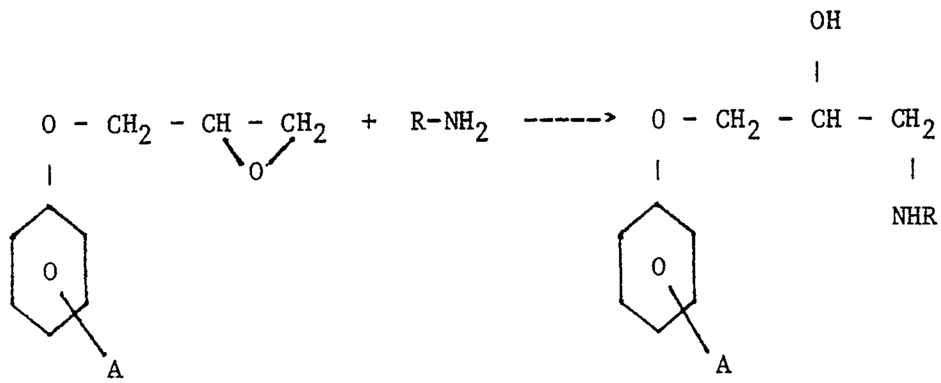

where A is a substituent group on the aromatic ring.

2,3-epoxypropyl ethers can be produced from the corresponding allyl ethers by chemical oxidation, using e.g. a peroxide, or by microbiological oxidation processes. One microbiological process is disclosed in Published European Patent Application No. 166527 and uses strains of microorganisms derived from the genera Nocardia , Brevibacterium , Corynebacterium , Pseudomonas , Rhodococcus , Arthrobacter and Micrococcus . In such known processes however we believe that the enzymes which cause oxidation of allyl ethers to the corresponding 2,3-epoxypropyl ethers are induced in the microorganisms during growth upon alkanes. We have found that such enzymes can affect substituents on the aromatic ring, creating unwanted by-products. It is also desirable that the productivity of the reactions used in the microbiological oxidation of allyl ethers to 2,3-epoxypropyl ethers should be increased.

The use of microorganisms containing the enzyme alkene monooxygenase, induced by growth upon alkenes, in the production of 1,2-epoxypropane and 1,2-epoxybutane has heen disclosed. We have now found that such microorganisms and enzymes derived from them can be employed in the production of 2,3-epoxypropyl ethers.

According to the present invention we provide a process for the production of an optically active 2,3-epoxypropyl ether in which a corresponding allyl ether having the general formula $X\text{-}O\text{-}CH_2\text{-}CH = CH_2$ (1), wherein X is as hereinafter defined, is treated under aerobic conditions with microorganism cells or an extract obtained therefrom containing the enzyme alkene monooxygenase, which enzyme is capable of oxidising an allyl ether to the corresponding 2,3-epoxypropyl ether and which enzyme has been induced in the cells by growth thereof on a culture medium containing an alkene, and the produced 2,3-epoxypropyl ether is isolated.

In the general formula $X\text{-}O\text{-}CH_2\text{-}CH = CH_2$ (1), X represents one of the following radicals:-

(a) phenyl and substituted phenyl radicals;
(b) substituted and unsubstituted α- and β- naphthyl radicals; and
(c) a benzyl radical.

When X is a substituted phenyl radical, suitable radicals include radicals having the formula:-

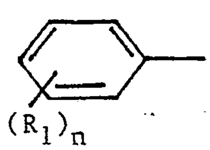 ;

wherein $R_1$ is one or more of the following groups:-

methyl, ethyl, n-propyl, iso-propyl, n-butyl iso-butyl, methoxy, ethoxy, n-propoxy, iso-propoxy, n-butoxy, iso-butoxy, t-butoxy, halogen, cyano, cyclopentyl, allyl, allyloxy, methoxyethyl and benzyloxy;
and n is an integer between 1 and 3, the $R_1$ groups being the same or different when n is 2 or 3.

Suitable substituted naphthyl radicals include radicals having the formula:-

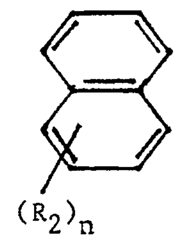
;

wherein $R_2$ is one or more of the following groups:-
methyl, ethyl, n-propyl, iso-propyl;
and n is an integer between 1 and 4, the $R_2$ groups being the same or different when n is 2 to 4.

Preferred allyl ethers for use as starting materials in the process of the invention include allyl phenyl ether, t-naphthyl allyl ether, 2-methylphenyl allyl ether, 4-n-butylphenyl allyl ether, 2,5-dimethylphenyl allyl ether, 2,3-dimethylphenyl allyl ether, 2,6-di-t-butyl-4-methylphenyl allyl ether, 2-allylphenyl allyl ether, 2-cyanophenyl allyl ether, 2-cyclopentylphenyl allyl ether, 2-methoxyphenyl allyl ether, 2-chlorophenyl allyl ether, 2-allyloxyphenyl allyl ether, 4-β-methoxyethylphenyl allyl ether, 4-butoxyphenyl allyl ether, 3-methylphenyl allyl ether, 2-chloro-5-methylphenyl allyl ether and benzyl allyl ether. A particularly preferred starting material is allyl phenyl ether. Generally the allyl ethers will be used individually as starting materials in the process of the invention but mixtures of allyl ethers may also be used.

Particularly useful microorganisms for use in the process of the invention are the novel strains NCIMB 40155 and NCIMB 40156 (also known as strains M26 and M156 respectively). Cultures of these strains have been deposited at the National Collections of Industrial and Marine Bacteria Ltd. (NCIMB), PO Box 31, 135 Abbey Road, Aberdeen, AB9 8DG, Scotland on 21 June 1989 under the terms of the Budapest Treaty on the International Recognition of Microorganisms for the Purposes of Patent Procedure. These novel microorganisms were derived originally from soil samples which had been suspended in a buffer in an atmosphere of ethene and propene. After 5 days of this treatment the soil was separated and the resulting cultures were subcultured repeatedly (i.e. 3 to 4 times) under ethene and propene. Standard separation techniques were used to produce biologically pure cultures.

In the process of the invention an allyl ether having the formula (1) as hereinbefore defined is treated with the enzyme alkene monooxygenase which enzyme is described by de Bont J.A.M. et al; (1979), FEMS Microbiol Lett., Vol. 3, pp 89-93. The enzyme may be present in microorganism cells, e.g. of the strains NCIMB 40155 or NCIMB 40156, strains of the genera Mycobacterium , Nocardia or Xanthobacter or in crude or purified extracts derived therefrom. The enzyme is preferably one which has heen induced in cells which are not capable of growth upon an alkane when such cells are grown upon a culture medium containing an alkene. Preferred alkenes for growth of the cells and induction of the enzyme are ethene and propene. When the enzyme is induced the strains can be grown initially on an alternative, non-enzyme inducing, carbon source such as glucose, acetate, lactate, and thereafter grown for a period of from 2 hours to 2 days on an alkene. An alternative method of growing cells to contain the enzyme is to use a carbon source such as glucose, acetate or lactate plus a suitable inducer of the enzyme other than ethene or propene.

The product of the process of the invention is a 2,3-epoxypropyl ether having the general formula;

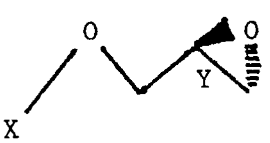

and having a chiral centre at Y. In epoxypropyl ethers which may be produced by the process which are very useful as intermediates X has one of the following structures:-

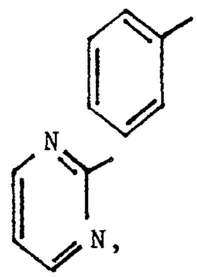 , 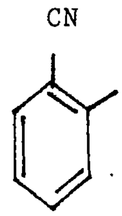 , 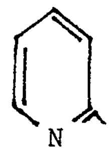 , 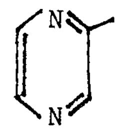 , , 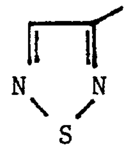 , 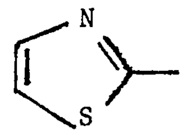 and 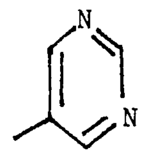 ,

Generally the enantiomer which is produced by the process of the invention is the S-enantiomer. Moreover the enantiomer which is produced is usually produced in greater than 80% enantiomeric excess. When the product is an intermediate it can then be subjected to one or a series of reactions to produce a desired final product. If the product produced in any subsequent reaction is a crystalline derivative this may be used to enhance the enantiomeric excess to a value closer to 100%.

When the process of the invention is carried out using the enzyme in microorganism cells it can be carried out in a number of alternative ways of which the following are the basic alternatives:-

(a) To carry out the process using from the start a culture medium containing a carbon source for growth of the cells containing the enzyme and the allyl ether starting material.

(b) To carry out the process in two stages in the same apparatus. In this alternative the microorganism is first grown in a culture medium containing a carbon source for growth with the allyl ether to be oxidised being added after a suitable interval at the beginning of a second stage.

(c) To carry out the process in two stages in different apparatus. In this alternative the microorganism is grown first in a first apparatus in a culture medium containing a carbon source for growth. At the start of the second stage the microorganism cells are separated from the growth culture medium and are transferred to a second apparatus containing the allyl ether to be oxidised in a suitable buffered medium. To this medium could also be added another oxidisable substrate to supply reducing power to the process. In this alternative the allyl ether could be present in a water-immiscible solvent.

Growth of the microorganism is carried out aerobically and in the presence of a culture medium containing suitable inorganic nutrients and any other materials such as yeast extract, corn steep liquor required for growth of the microorganism.

The use of the process of the invention enables an optically active 2,3-epoxypropyl ether to be produced with a high degree of productivity to a high stereopecificity and with a low incidence of side products.

The invention is illustrated by the following Examples, the results of which are shown in Figures 1 and 2 of the drawings:-

In the drawings:-

Figure 1 is a graph of concentration (mM) against time (a) showing the transformation of allyl phenyl ether (■,S) into 1,2-epoxy-3-phenoxypropane (●,P) by microorganism M26 in Example 1; and

Figure 2 is a graph of concentration (mM) against time (b) showing the transformation of allyl phenyl ether (■,S) into 1,2-epoxy-3-phenoxypropane (●,P) by microorganism M156 in Example 2.

## EXAMPLE 1

Transformation allyl phenyl ether to 1,2-epoxy-3-phenoxypropane using an ethene grown microorganism.

The microorganism M26 (NCIMB 40155) was grown in two litre Erlenmeyer flasks containing 400 ml of mineral salts medium, the composition of which is given in Table 1. The carbon source was 5% v/v ethene introduced by syringe through a septum. The culture inoculum (4% v/v) consisted of cells pre-adapted to growth on ethene. The culture was incubated at 30° C for 72 hours, after which time the cells were harvested by centrifugation and washed in cold 25 mM phosphate buffer at pH 7.0. The cells were resuspended to give an optical density, measured at 600 nm, of 10-15 units. Into a 100 ml screwed topped conical flask was placed 12 ml of washed cell suspension. The suspension was preincubated at 30° C for 5

min and then the bioconversion was begun hy addition of allyl phenyl ether to a final concentration of 2 mM. Samples (50 µl) were withdrawn at intervals and extracted with an equal volume of ice-cold ether containing 1-heptadecene as an internal standard. The mixture was separated by centrifugation and a sample from the ether layer analysed by gas chromatography on a 10% Carbowax 20M or Chromosorb WHP (1.8 m x 4 mmid) at 190°C, with nitrogen carrier gas at 40 ml $min^{-1}$. The results of a 12 hour bioconversion are shown in Fig. 1. The final concentration of 1,2-epoxy-3-phenoxypropane achieved represented a yield of 80% (g product/g allyl ether added).

For analysis of the enantiomeric composition of the product the above method was scaled up by a factor of 28. After 10 hours incubation with allyl phenyl ether the whole broth was extracted twice with equal volumes of ice-cold ether (not containing internal standard). The combined ether layers were washed once with phosphate buffer (pH 8), dried over $Na_2SO_4$, and then concentrated by rotary evaporation.

The concentrated extract was examined for enantiomeric composition by chiral HPLC; column - Chiracel OD 25 cm x 0.4 cm id); eluent - iso propyl alcohol/hexane 40/50 (v/v); flow rate -0.5 ml $min^{-1}$; detector - 280 nm; injection volume - 20 µl. The enantiomeric excess was measured at 93%, with the S-enantiomer predominating.

Table 1

| Composition of medium used for the cultivation of organism M26 | |
|---|---|
| Constituent | Concentration ($gl^{-1}$) |
| $KNO_3$ | 2.4 |
| $MgSO_4.7H_2O$ | 0.2 |
| $CaCl_2.2H_2O$ | 0.002 |
| $FeSO_4.7H_2O$ | 0.001 |
| $ZnSO_4.7H_2O$ | $7 \times 10^{-5}$ |
| $MnCl_2.4H_2O$ | $1.3 \times 10^{-5}$ |
| $H_3BO_3$ | $1 \times 10^{-5}$ |
| $CuSO_4.5H_2O$ | $5 \times 10^{-6}$ |
| $NaMoO_4.H_2O$ | $1 \times 10^{-7}$ |
| Phosphate buffer | Final |
| Sterile stock, (pH 7.2 added after autoclaving) | 50 mM |

EXAMPLE 2

Transformation of allyl phenyl ether to 1,2-epoxy-3-phenoxypropane by a propene grown microorganism

The microoganism M156 was grown as described in Example 1. The carbon source was 10% (v/v) propene. All other conditions and manipulations were as described in Example 1. Figure 2 shows the result of a 5 hour bioconversion. The measured enantiomeric excess of the product was 93%, with the S-enantiomer predominating.

## Claims

1. A process for the production of an optically active 2,3-epoxypropyl ether in which a corresponding allyl ether having the general formula $X\text{-}O\text{-}CH_2\text{-}CH = CH_2$ (1), wherein X is as hereinbefore defined, is treated under aerobic conditions with microorganism cells or an extract obtained therefrom containing the enzyme alkene monooxygenase, which enzyme is capable of oxidising an allyl ether to the corresponding 2,3-epoxypropyl ether and which enzyme has been induced in the cells by growth thereof on a culture medium containing an alkene, and the produced 2,3-epoxypropyl ether is isolated.

2. A process according to claim 1 wherein the allyl ether has a general formula in which X is a phenyl radical or a substituted phenyl radical, said substituted phenyl radical having the formula:-

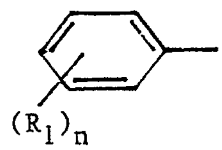

;

$R_1$ being one or more of the following groups:-
methyl, ethyl, n-propyl, iso-propyl, n-butyl, iso-butyl, methoxy, ethoxy, n-propoxy, iso-propoxy, n-butoxy, iso-butoxy, t-butoxy, halogen, cyano, cyclopentyl, allyl, allyloxy, methoxyethyl and benzyloxy;
and n being integer between 1 and 3, the $R_1$ groups being the same or different when n is 2 or 3.

3. A process according to claim 1 wherein the allyl ether has a general formula in which X is a substituted or unsubstituted $\alpha$- or $\beta$- naphthyl radical, said substituted naphthyl radical having the formula:-

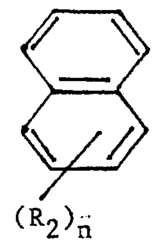

;

$R_2$ being one or more of the following groups:-
methyl, ethyl, n-propyl, iso-propyl;
and n being an integer between 1 and 4, the $R_2$ groups being the same or different when n is 2 to 4.

4. A process according to any one of claims 1 to 3 wherein the microorganism cells belong to strain M26 (NCIMB 40155) or M156 (NCIMB 40156).

5. A process according to any one of claims 1 to 4 wherein the enzyme is one which has been induced in cells which are not capable of growth upon an alkane when such cells are grown upon a culture medium containing an alkene.

6. A process according to any one of claims 1 to 5 wherein the enzyme has been induced in the cells by growth thereof on a culture medium containing ethene or propene.

7. A process according to any one of claims 1 to 6 wherein the treatment is initiated in a culture medium containing a carbon source for growth of the cells containing the enzyme and the allyl ether starting material.

8. A process according to any one of claims 1 to 6 which comprises a first stage in which the microorganism is grown in a culture medium containing a carbon source for growth and, after an interval, a second stage in which the allyl ether to be oxidised is added.

9. A process according to claim 8 wherein the first stage is carried out in a first vessel and, between the first and second stages, the microorganism cells are separated from the growth culture medium and are transferred to a second vessel in which the second stage is carried out.

10. A process according to claim 9 wherein, in the second stage, the allyl ether is present in a water-immiscible solvent.

*Fig. 1.*

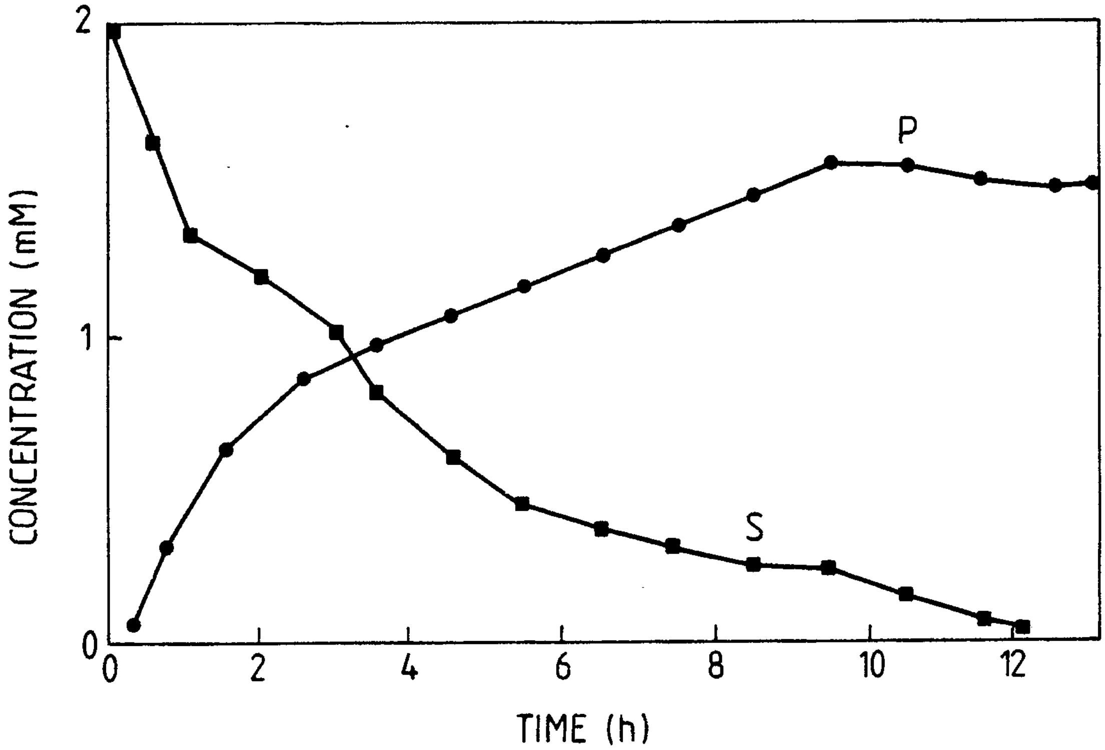
CONCENTRATION (mM)
2
1
0
0
2
4
6
8
10
12
TIME (h)
P
S

*Fig. 2.*

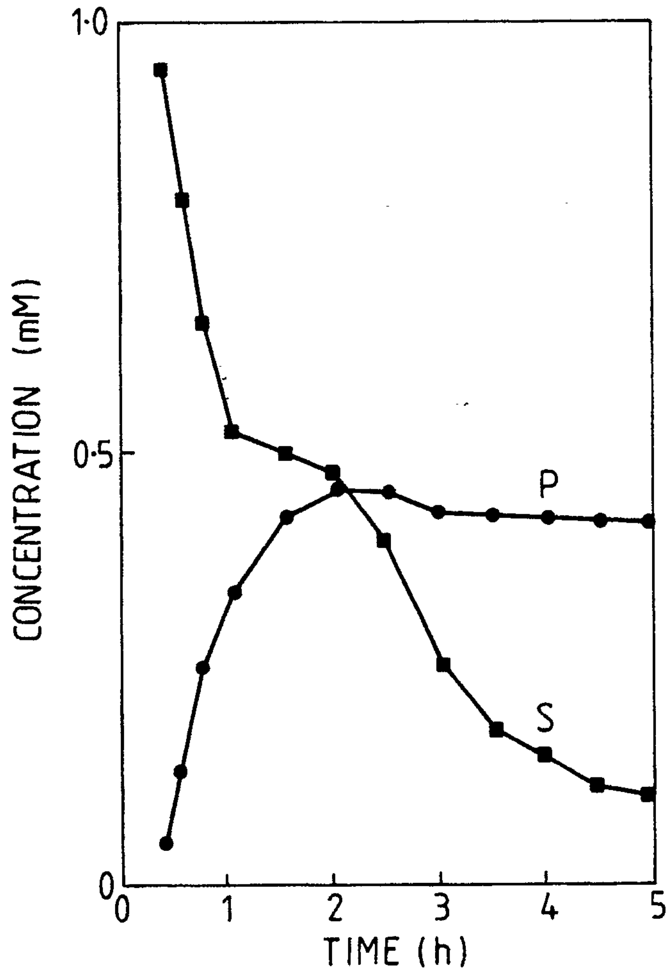
CONCENTRATION (mM)
1·0
0·5
0
0
1
2
3
4
5
TIME (h)
P
S